(19)

**Europäisches Patentamt**
**European Patent Office**
**Office européen des brevets**

(11) **EP 4 512 412 A1**

(12) **EUROPEAN PATENT APPLICATION**
published in accordance with Art. 153(4) EPC

(43) Date of publication:
26.02.2025 Bulletin 2025/09

(51) International Patent Classification (IPC):
A61K 38/17 (2006.01)     A61P 25/00 (2006.01)
A61P 25/24 (2006.01)     A61P 25/22 (2006.01)
C07K 14/705 (2006.01)     C12N 15/12 (2006.01)
C12N 15/70 (2006.01)     C12N 15/85 (2006.01)
C12N 5/10 (2006.01)     C12N 1/21 (2006.01)

(21) Application number: 23791315.7

(22) Date of filing: 20.04.2023

(86) International application number:
PCT/CN2023/089490

(87) International publication number:
WO 2023/202663 (26.10.2023 Gazette 2023/43)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 21.04.2022 CN 202210427358

(71) Applicant: Shenzhen Chenyang Biological
Technology Co., Ltd
Shenzhen, Guangdong 518000 (CN)

(72) Inventors:
• TAN, Zhen
  Shenzhen, Guangdong 518000 (CN)
• LI, Tao
  Shenzhen, Guangdong 518000 (CN)
• LI, Shupeng
  Shenzhen, Guangdong 518000 (CN)

(74) Representative: Kailuweit & Uhlemann
Patentanwälte
Partnerschaft mbB
Bamberger Straße 49
01187 Dresden (DE)

Remarks:
The complete document including Reference
Table(s) and the Sequence Listing(s) can be
downloaded from the EPO website

(54) **POLYPEPTIDE AND USE THEREOF**

(57) Provided are a polypeptide and a use thereof. Provided is a use of a polypeptide in preparation of a medication for treating and/or preventing PTSD, depression, or anxiety disorder, thereby providing a treatment strategy for PTSD, depression, or anxiety disorder. Provided are a polypeptide, a complex, a nucleic acid molecule, an expression vector, and a host cell.

FIG. 7

**Description**

**Technical Field**

The present invention relates to the field of research and development of medicament, and specifically relates to medical use of a polypeptide.

**Background**

Posttraumatic stress disorder (PTSD) refers to a mental disorder that occurs at a delayed time and lasts for a prolonged period in an individual who has experienced, witnessed, or encountered with one or more of actual death, threat of death, serious injury, or threat of physical integrity involving the individual himself or herself or another person. Studies have shown that PTSD is induced by a failed response to stress at the time of trauma, which leads to a series of changes, including hormonal and neurochemical changes, as well as a structural defect in the brain. According to a hypothesis, cortisol dysregulation at the time of trauma may delay the effect of norepinephrine on synapses in the peripheral or central nervous system, affecting the memory consolidation of events. In contrast, adrenergic activation facilitates learning at a low cortisol level. In addition, physical and chemical dysregulations in PTSD may affect the serotonergic and dopaminergic mechanisms.

In order to provide more abundant and better treatment strategies and better elucidate the pathogenesis, there is still a strong need to further explore and identify the pathogenesis and therapeutic target(s) of PTSD.

**Summary of the Invention**

In order to achieve the above purpose(s), the inventors have conducted researches and found that the D2 receptor in dopamine (DA) receptors and the 5-HT2CR in 5-hydroxytryptamine (5-HT) receptors have a dimerization interaction. Further results showed that the dimer could serve as a target for treating PTSD, thereby accomplishing this invention.

Specifically, DA receptors are receptors that are located within organisms and function through their corresponding membrane receptors. They can be divided into five types: D1, D2, D3, D4, and D5. The D2 receptor (D2R) is widely expressed in the brain.

5-HT receptors are a group of G protein-coupled receptors and ligand-gated ion channels that exist in the central part of the central nervous system and the peripheral region of the peripheral nervous system. They can be divided into seven subfamilies: 5-HT1, 5-HT2, 5-HT3, 5-HT4, 5-HT5, 5-HT6, and 5-HT7. The 5-HT2 receptors comprise three subtypes of A, B, and C, namely, the 5-HT2A, 5-HT2B, and 5-HT2C receptor proteins. Among them, 5-HT2CR is widely expressed throughout brain, including the limbic-midlimbic cortex as well as regions in striatum, such as VTA, NAc, PFC, amygdala, hippocampus, and dorsal striatum, primarily in a presynaptic location.

Upon recognizing the existence of the D2R/5-HT2CR dimer, the inventors further found from researches that D2R could interact, for example, through its K226-L240 region (hereinafter referred to as the "KL peptide", the sequence thereof is as shown in SEQ ID NO: 1), with 5-HT2CR. Subsequently it was confirmed using an animal model that the interaction of the D2R/5-HT2CR complex was interfered by the KL peptide, which thereby has an effect of treating PTSD.

Accordingly, in a first aspect, the present invention provides a polypeptide having an amino acid sequence as shown in SEQ ID NO: 1. Further, also provided is use of the polypeptide of the present invention in preparation of a medicament for treating and/or preventing PTSD.

In some embodiments, the polypeptide of the present invention may have a length of 15 to 30 aa, for example, 16 aa, 17 aa, 18 aa, 19 aa, 20 aa, 21 aa, 22 aa, 23 aa, 24 aa, 25 aa, 26 aa, 27 aa, 28 aa, or 29 aa.

In some embodiments, the polypeptide of the present invention may be derived from D2R. The D2R from which the polypeptide is derived can be interrogated by searching a bioinformatics database (such as Genbank, EMI, DDBJ, etc.), and the sequence of the polypeptide can be confirmed based on this. For example, in the case where the polypeptide is derived from the D2R (Genbank Accession No. CAB56463), in addition to the sequence as shown in SEQ ID NO: 1 (i.e., positions 226 to 240 of the amino acid sequence of CAB56463), the polypeptide can further include one or more additional amino acid residue(s). For example, a polypeptide sequence from positions 225 to 240 of CAB56463, in which case it has 16 amino acid residues.

In some embodiments, the D2R is derived from primates.

In a specific embodiment, the D2R is derived from human.

In some embodiments, the polypeptide of the present invention consists of up to 30 consecutive amino acid residues derived from human D2R and comprises the sequence as shown in SEQ ID NO: 1.

In an exemplary embodiment, the polypeptide of the present invention consists of up to 29, 28, 27, 26, 25, 24, 23, 22, 21, 20, 19, 18, 17, or 16 consecutive amino acid residues derived from human D2R and comprises the sequence as shown in SEQ ID NO: 1.

In a specific embodiment, the amino acid sequence of the D2R derived from human is as shown in SEQ ID NO: 2.

As used herein, the term "treating" refers to inducing in a patient a desired or beneficial effect which may include reducing the frequency or severity of one or more symptoms of a disease, or suppressing or inhibiting the further progression of a disease, condition, or disorder.

As used herein, the term "preventing" refers to preventing or delaying the onset of a disease or its clinical or subclinical symptom(s).

In addition, the inventor found from further researches that the polypeptide of the present invention is also useful for controlling depression and anxiety disorder.

Accordingly, provided is use of the polypeptide of the present invention in preparation of a medicament for treating and/or preventing depression. Also provided is use of the polypeptide of the present invention in preparation of a medicament for treating and/or preventing anxiety disorder.

As used herein, "depression" refers to a predominantly depressed state of mind that is disproportionate to one's situation and can range from feeling down to extreme grief; and in certain cases, and even to the onset of a stupor.

As used herein, "anxiety disorder" refers to a neurosis mainly characterized by anxious emotions.

The polypeptide of the present invention works through a new pathogenesis mechanism (disrupting the D2R/5-HT2CR complex) to exert an anti-PTSD effect, indicating an excellent clinical development value. In addition, the polypeptide of the present invention can also exert anti-depression and anti-anxiety effects.

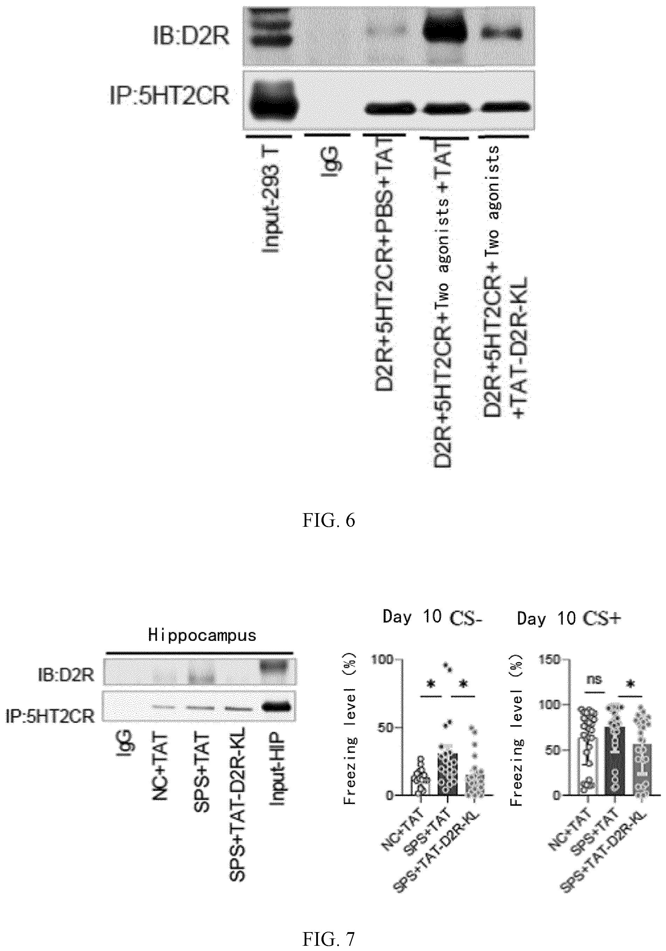

It is noteworthy that as demonstrated by Examples 4 and 5 below, as well as FIGs. 6, 10 and 11, the polypeptide of the present invention disrupted the interaction of the D2R/5-HT2CR complex without significantly affecting the expression of DA or 5-HT. In other words, the polypeptide of the present invention works by specifically disrupting the interaction of the D2R/5-HT2CR complex.

In a second aspect, the present invention provides a nucleic acid molecule encoding the polypeptide of the present invention.

In an alternative embodiment, the nucleic acid molecule of the present invention is a reverse-complement sequence of the coding sequence of the polypeptide of the present invention.

In an exemplary embodiment, the nucleic acid molecule of the present invention is as shown in SEQ ID NO: 4.

Further, also provided is use of the nucleic acid molecule of the present invention in preparation of a medicament for treating and/or preventing PTSD, depression and/or anxiety disorder.

The nucleic acid molecule of the present invention can be used to produce the polypeptide of the present invention, and according to the expression system employed, a person skilled in the art can appropriately adjust the sequence of the nucleic acid molecule, for example, based on the codon preference for the selected expression system.

In a third aspect, the present invention provides an expression vector comprising the nucleic acid molecule of the present invention. Further, also provided is use of the expression vector of the present invention in preparation of a medicament for treating and/or preventing PTSD, depression and/or anxiety disorder.

A variety of known methods can be used to insert the nucleic acid molecule of the present invention into an expression vector. For example, the nucleic acid molecule can be inserted into an appropriate restriction endonuclease site. The standard techniques for cloning, isolation, amplification and purification, as well as enzymatic reactions involving a DNA ligase, a DNA polymerase, a restriction endonuclease, or the like and the various separation techniques in the operations are known and commonly used by a person skilled in the art.

In a fourth aspect, the present invention provides a host cell comprising the nucleic acid molecule or the expression vector of the present invention. Further, also provided is use of the host cell of the present invention in preparation of a medicament for treating and/or preventing PTSD, depression and/or anxiety disorder.

A variety of expression vectors and host cells from expression systems such as a prokaryotic and a eukaryotic expression system can be used to produce the polypeptide of the present invention. In the following description, the mammalian expression system is taken as an example, wherein the host cell may include the COS-7 cell line of monkey kidney fibroblasts and other cell lines capable of expressing a compatible vector, such as the C127, 3T3, CHO, Hela, and BHK cell lines. A mammalian expression vector should comprise a replication origin, a suitable promoter and enhancer, along with any required ribosome binding site, polyadenylation site, splice donor site and splice acceptor site, transcription terminator sequence, and 5'-non-transcribed flanking sequence. DNA sequences derived from, for example, SV40 splicing and polyadenylation sites can be used to provide a required non-transcriptional genetic element. The expression vector can be introduced into a host cell by a variety of methods familiar to a person skilled in the art, including but not limited to, for example, calcium phosphate transfection, DEAE-dextran-mediated transfection, or electroporation.

In a fifth aspect, the present invention provides a complex comprising the polypeptide of the present invention and a (transport) carrier for permeating the blood-brain barrier linked to the polypeptide. The complex of the present invention is intended to make the polypeptide of the present invention more suitable for delivery to humans or animals for treating and/or preventing PTSD, depression and/or anxiety disorder.

In an exemplary embodiment, the carrier used for permeating the blood-brain barrier can be one or more of HIV-1 Tat protein, insulin, cationized albumin, monoclonal antibody (mAb) against rat transferrin receptor (OX26), human insulin receptor murine mAb (HIRMAb), Penetratin, transduction domain of Tat protein, Pep-1 peptide, $S4_{13}$-PV, Magainin 2, and Buforin 2. For example, the TAT transduction domain can transduce across the membrane into a cell, and the amino acids thereof are YGRKKRRQRRR (as shown in SEQ ID NO: 5).

The polypeptide of the present invention can be linked to a carrier for permeating the blood-brain barrier by an appropriate linkage technique. An exemplary linkage technique can be the avidin-biotin technique, the polyethylene glycol (PEG)-based space arm technique, and the fusion protein technique, etc. For example, when the transduction domain of the HIV-1 Tat protein is used as a carrier for permeating the blood-brain barrier, the fusion protein technique can be used to directly link the polypeptide of the present invention to the transduction domain of the Tat protein.

In some embodiments, when the fusion protein technique is used, a linker may also be used to link the polypeptide of the present invention to a carrier for permeating the blood-brain barrier. An exemplary linker can be a flexible linker containing glycine, such as G, GSG, GSGGSG, GSGGSGG, GSGGSGGG, GGGGSGGG, GGGGS and SGG, etc.

In a sixth aspect, the present invention provides a method for treating or preventing PTSD, depression and/or anxiety disorder in a subject, comprising:

administering to the subject an effective amount of the polypeptide or the complex of the present invention.

The above method is achieved through the polypeptide of the present invention which specifically disrupts the interaction of the D2R/5-HT2CR complex.

The term "effective amount" or "therapeutically effective amount" refers to the amount of an active agent sufficient to induce a desired biological outcome. The outcome can be reduction in the signs, symptoms, or causes of a disease, or any other desired change in a biological system. The term "therapeutically effective amount" used herein refers to any amount of a preparation that induces substantial alleviation of a disease when it is repeatedly administered to the affected area over a period of time. This amount can vary depending on the condition being treated, the progression of the condition, and the type and concentration of the preparation used. A person skilled in the art can determine the appropriate amount through conventional experiments.

The terms "subject", "individual", and "patient" are used interchangeably herein to refer to a vertebrate, preferably a mammal, and more preferably a human being. The mammal includes, but is not limited to, a murine, a monkey, a human being, a farm animal, and a pet. A tissue, cell, and progeny of a biological entity obtained or cultured *in vitro* are also included.

In a seventh aspect, provided is the polypeptide or the complex of the present invention for use in the treatment and/or prevention of PTSD, depression and/or anxiety disorder.

## Description of the Drawings

FIG. 1 shows the results of Western blotting analysis of co-immunoprecipitation for the mouse hippocampal tissue;

FIG. 2 shows the results of Western blotting analysis of co-immunoprecipitation in the SPS mouse model;

FIG. 3 shows the locations of the fragments referred to in Example 3 on D2R;

FIG. 4 shows the results of Western blotting analysis of GST pulldown assay for the mouse hippocampal tissue using the fragments in Example 3;

FIG. 5 shows the experimental design flow chart of Example 4;

FIG. 6 shows the results of Western blotting analysis of co-immunoprecipitation for the polypeptide-treated 293T cells;

FIG. 7 shows the results of Western blotting analysis of co-immunoprecipitation for the hippocampal tissue of the polypeptide-treated SPS mice (left), and effect of the polypeptide treatment on freezing level of the SPS mice (right);

FIG. 8 shows the effect of the polypeptide on corticosterone level in the hippocampus of SPS mice;

FIG. 9 shows the effect of the polypeptide on cortisol level in the hippocampus and the cortex of SPS mice;

FIG. 10 shows the effect of the polypeptide on DA level in the hippocampus and the cortex of SPS mice;

FIG. 11 shows the effect of the polypeptide on 5-HT level in the hippocampus and the cortex of SPS mice;

FIG. 12 shows the results of OFT, TST, FST, and SPT analysis of polypeptide-treated CRS mice in Example 6, with $*P<0.05$, $**P<0.01$, and $***P<0.001$;

FIG. 13 shows the results of OFT analysis of polypeptide-treated CRS mice in Example 7, with $*P<0.05$, and $**P<0.01$; and

FIG. 14 shows the results of EPM analysis of polypeptide-treated CRS mice in Example 7, with $**P<0.01$.

## Specific Embodiments

Throughout the Description, unless otherwise specifically stated, the terms used herein shall be construed as having the meaning commonly used in the art. Therefore, unless otherwise defined, all technical or scientific terms used herein have the same meaning as generally understood by a person skilled in the art to which the present invention belongs; if there is a contradiction, the present Description shall prevail.

The embodiments of the present invention will be described in detail below in reference to the Examples, and the advantages and various effects of the present invention will thereby become clearer. A person skilled in the art should understand that these embodiments and Examples are used to illustrate rather than limit the present invention.

Where specific conditions were not indicated in the Examples, the conventional conditions or the conditions recommended by the manufacturer were used. The reagent or instrument used without indication of the manufacturer was a conventional product which was commercially available.

Experimental Animals

The Balb/c mice were purchased from the Guangdong Medical Laboratory Animal Center in China. They were male, 12-14 weeks old, and raised at 18-22°C in a 12 h light/12 h dark cycle. They had free access to food and tap water throughout the process.

The adult C57BL/6J mice (male, 12-14 weeks old) were raised at 18-22°C in a 12 h light/12 h dark cycle. They had free access to food and tap water throughout the process.

Statistical Analysis

The results were expressed as mean + s.e.m. Statistical analysis was performed using the Graphpad Prism 8 software. One-way analysis of variance (ANOVA) and post-hoc test were used to evaluate the potential difference between mean values. Unless otherwise stated, the independent samples t-test was used to compare any two given groups throughout the research. Unless otherwise stated, there was significance if $P < 0.05$ in a test.

Example 1 Confirmation of formation of D2R/5-HT2CR complex *in vivo*

Co-immunoprecipitation was performed using protein samples (with 100-500 μg protein) from the hippocampal tissue of the Balb/c mice. Mouse monoclonal antibodies against both 5-HT2CR and D2R, D2R, or 5-HT2CR were used for precipitation with 25 μl of the slurry of protein A/G + agarose beads (SantaCruz Biotechnology, sc-2001).

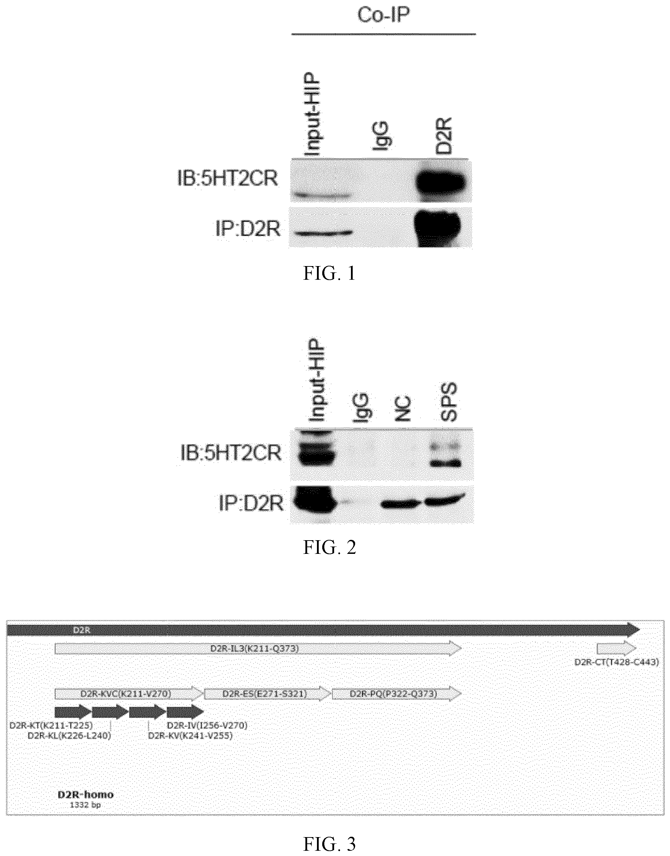

Western blotting analysis after precipitation: Denatured (at 100°C for 10 min) proteins were separated on an 8% SDS-PAGE gel, then transferred to a nitrocellulose membrane, which was then blocked with skim milk in TBST (Tris-buffered saline with 0.1% Tween 20). Next, incubation was carried out with the primary antibodies at 4°C overnight. Finally, treatment with an HRP-conjugated secondary antibody were performed for 1 h, signals were detected using the ECL UltraSignal chemiluminescence kit (4ABiotech, cat. no. 4AW011-500), and the bands were analyzed for densities using the Image Lab software. Results were shown in FIG. 1.

As could be seen from FIG. 1, the antibodies against 5-HT2CR pulled down D2R in the hippocampal tissue, confirming the conjugation of D2R to 5-HT2CR.

Example 2 Verification of the pathological significance of the complex in PTSD

To determine whether such conjugation was associated with PTSD, we established a single prolonged stress (SPS) model as follows:

The SPS model was established with male Balb/c mice. The modified SPS protocol was used to comprise a series of stress exposure: 2 hours of restraint stress (inside a 50-mL plastic tube having several air holes), 3 min of rest, 12 min of forced swimming in a transparent glass tank (at about 24°C), 15 min of rest (drying under a warming lamp), 20 min of predator (rat) odor exposure, followed by ether anesthesia (until the mice completely lost consciousness). Thereafter, the mice are transferred back to their cages with fresh bedding. The control mice were housed in groups and received no stress exposure. After the model establishment, all mice were raised undisturbed for one week.

Using the established SPS mouse model, the interaction of D2R and 5-HT2CR in this model was analyzed by the co-immunoprecipitation and the Western blotting methods as described in Example 1, and results were shown in FIG. 2.

As could be seen in FIG. 2, a significant interaction between D2R and 5-HT2CR was found in the SPS model, which confirmed the dimerization of them.

Example 3 Identification of the binding site of the complex

In order to identify the binding site of the D2R/5-HT2CR complex, full-length D2R cDNA clone (Genbank Accession No. X51645) was first amplified to obtain the cDNA fragments of the CT region of D2R (D2R-CT, T428-C443) and the third intracellular loop (IL3) region of D2R (D2R-IL3, K211-Q373). These fragments were subcloned into the the BamH1/EcoR1 or BamH1/Xho1 site of pGEX-4T-3 plasmids (YouBio no: VT1255). The initiation methionine residue and the termination codon were incorporated when appropriate. All constructs were resequenced to confirm that the splicing fusion was properly achieved. *E. coli* BL21 viable cells (KangTi Life Technology, no: KTSM104L) were used for expression, and purification was performed to obtain the GST fusion protein containing the IL3 region of D2R (GST-D2R-IL3) and the GST fusion protein containing the CT region of D2R (GST-D2R-CT) from the bacterial lysates. The specific locations of the coding sequences for the CT region and the IL3 region on D2R were as shown in FIG. 3.

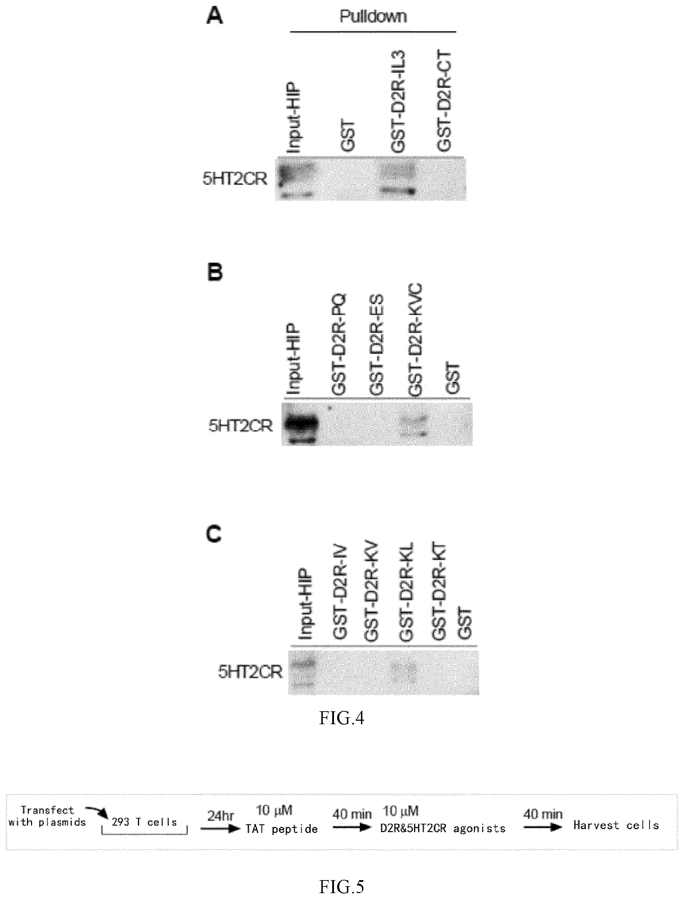

The dissolved mouse hippocampal tissue extract (with 500 μg protein) was diluted with 1 × PBS / 1% Triton X-100 and subsequently incubated with 20 μl of protein-GST-resin saturated with only GST protein or 15 μg of the GST fusion protein described above overnight at 4°C. Beads were washed with 1 × PBS / 1% Triton X-100 for 1-8 times. The bound proteins were eluted with SDS-PAGE Protein Loading Buffer (YeSen, no: 20315ES05), separated with SDS-PAGE, and subjected to Western blotting with their respective antibodies. Results were as shown in panel A of FIG. 4.

As could be seen from panel A of FIG. 4, the IL3 region of D2R could pull down the 5-HT2CR.

Next, in order to further explore the sequence/site for interaction between D2R and 5-HT2CR, we divided the IL3 region into the KVC (K211-V270) region of D2R, the ES (E271-S321) region of D2R, and the PQ (P322-Q373) region of D2R, with their specific locations on D2R shown in FIG. 3.

According to the method in this Example, pulldown analysis was performed using the GST fusion protein containing the KVC region of D2R (GST-D2R-KVC), the GST fusion protein containing the ES region of D2R (GST-D2R-ES), and the GST fusion protein containing the PQ region of D2R (GST-D2R-PQ), and the Western blotting results were shown in panel B of FIG. 4.

As could be seen from panel B of FIG. 4, the KVC region of D2R could pull down the 5-HT2CR.

Further, we divided the KVC region into the KT (K211-T225) region of D2R, the KL (K226-L240) region of D2R, the KV (K241-V255) region of D2R, and the IV (I256-V270) region of D2R, with their specific locations on D2R shown in FIG. 3.

According to the method in this Example, pulldown analysis was performed using the GST fusion protein containing the KT region of D2R (GST-D2R-KT), the GST fusion protein containing the KL region of D2R (GST-D2R-KL), the GST fusion protein containing the KV region of D2R (GST-D2R-KV), and the GST fusion protein containing the IV region of D2R (GST-D2R-IV), and the Western blotting results were shown in panel C of FIG. 4.

As could be seen from panel C of FIG. 4, D2R could associate with 5-HT2CR with an affinity from mouse hippocampal tissue through the KL polypeptide (K226-L240), indicating that D2R could interact with 5-HT2CR via its K226-L240 region.

Example 4 Confirmation of reduction of the D2R/5-HT2CR dimerization by the polypeptide

To explore whether deconjugation of 5HT2CR from D2R by the competing KL polypeptide could reverse their dimerization, the C-terminus of the KL (K226-L240) region of D2R was fused to the transduction domain of HIV-1 Tat protein (as shown in SEQ ID NO: 5, hereinafter referred to as "TAT") to obtain a fusion protein named TAT-D2R-KL, which could permeate the blood-brain barrier.

Next, the 293T cells were treated with TAT-D2R-KL in the presence of 5-HT2CR and D2R agonists, while treatment with TAT was used as a control. The specific process was shown in FIG. 5, where the human 293T cell line was cultured in a high-glucose modified Eagle's medium (DMEM) supplemented with 10% fetal bovine serum (FBS). The cells were maintained in an atmosphere of 95% air and 5% carbon dioxide in an incubator at 37°C. When the cells were grown to a density of 70%, transfection was performed using 5-HT2CR and D2R plasmids in a ratio of 1:1 (purchased from Addgene and Vigenebio (Cat#66411 and CH805293)).

The treated cells were analyzed according to the method in Example 1, and the results were shown in FIG. 6. It could be seen that the treatment with TAT-D2R-KL significantly reduced the interaction between 5-HT2CR and D2R.

Example 5 Evaluation of the anti-PTSD effect of the polypeptide

In order to evaluate the anti-PTSD effect of the polypeptide of the present invention, an SPS mouse model was established according to Example 2.

The fear conditioning, safety learning, and recall tests were carried out in two contexts (the fear conditioning and safety learning tests were carried out in context A; and the recall test was carried out in context B). The shapes and smells provided in contexts A and B were different. Indoor floors were cleaned with 1% acetic acid or 75% ethanol prior to testing. A camera was positioned on the top of the experimental testing chamber to record the freezing behavior. After the experiment was completed, the freezing level was manually calculated based on the recorded video. The criterion for a freezing behavior is that if no movement other than normal breathing of a mouse was detected within 2 seconds, then the mouse was considered freezing. The experimental mice received 4 times of CS+ and CS- stimulations (30 seconds, 60 dB; CS+: 50ms pips tone (3 kHz); CS-: white noise). On day 2, the test of fear conditioning was performed by pairing CS+ with US (2 seconds of foot shock at 0.8 mA) (3 times of CS+/US pairing with inter-trial interval (ITI) of 90 seconds). On day 3, the mice were tested under context B with 5 or 15 times of CS+ and CS-, respectively. On day 9 when the safety learning was carried out, the SPS mice were treated with TAT-D2R-KL for 1.5 h (with 3 nM TAT peptide administered intraperitoneally, 100 $\mu$l/10 g mouse), and then received CS+ and CS- in a pseudo-random manner (ITI: 90 seconds) in context A, wherein CS+ was paired with US while CS- was never reinforced with foot shock. On day 10, the polypeptide-treated mice were tested again in context B.

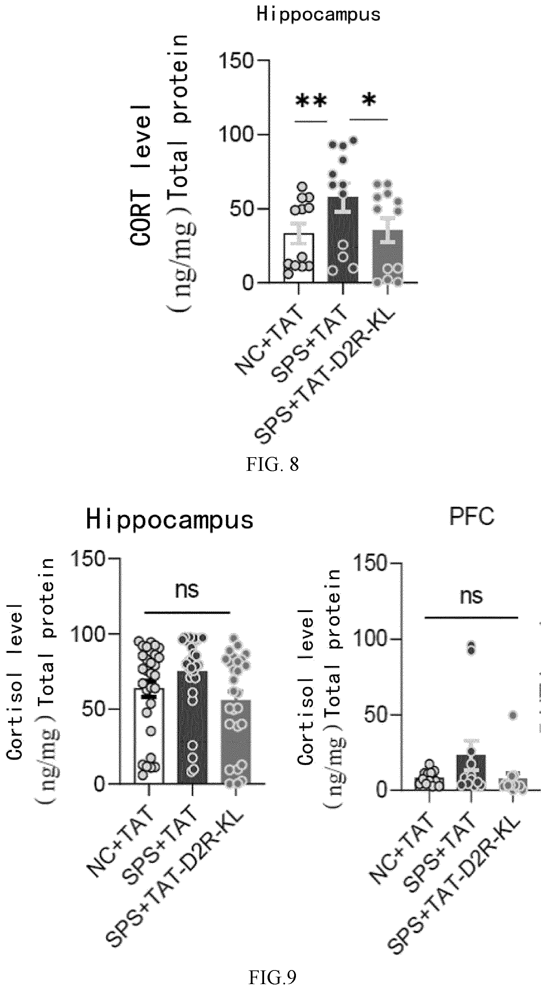

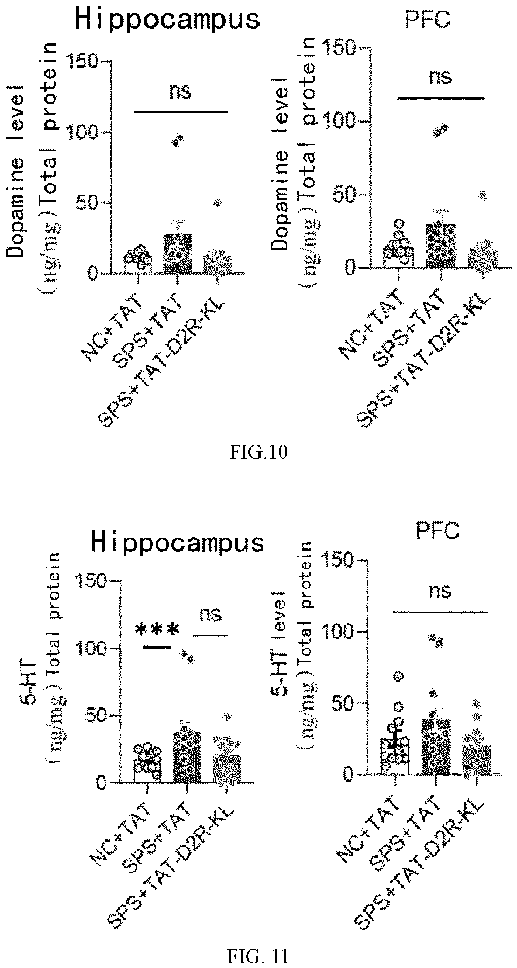

Interestingly, with the decrease of the the interaction between 5-HT2CR and D2R, the TAT-D2R-KL treatment reduced the freezing level in SPS mice (FIG. 7). In addition, TAT-D2R-KL significantly reduced the elevated corticosterone level in the hippocampus of the SPS mice (FIG. 8). However, TAT-D2R-KL did not significantly change the levels of cortisol (FIG. 9) and DA (FIG. 10) in the hippocampus and cortex of the SPS mice, and did not reduce the upregulated 5-HT in the hippocampus of the SPS mice (FIG. 11).

The above results confirmed that the polypeptide of the present invention achieved an anti-PTSD effect by deconjugating the D2R/5-HT2CR complex.

Example 6 Evaluation of the anti-depression ability of the polypeptide using a medicament evaluation model

In order to further evaluate the ability of the polypeptide as an anti-depression medicament, CRS and CMS mouse models were established as follows.

CRS:

The C57BL/6J mice were horizontally fixed for 6 h (from 10 to 16 o'clock) in a first acrylic restraint which is cylindrical and flat-bottomed ($25 \times 90$ mm) every day for 2 weeks. The filter had several slots which could adapt to the size of each mouse to firmly restrain the mouse and inhibit body movements of the limb and trunk without causing pain. Immediately after being restrained, the mice were sent back to their cages. Unrestrained mice (control) stayed in their cages when no CRS procedure was performed. Neither the control mice nor the CRS mice could have access to food and water during the CRS exposure.

CMS:

The C57BL/6J mice were subjected to various stress including restraint (for 4 h), cage tilting (for 45 times, with 12 h each time), light-dark cycle reversal (once), flash (for 12 h), and dirty cage (for 2 times, with 14 h each time). The protocol lasted 6 weeks. Control mice were not subjected to corresponding stress.

The mice were treated with TAT or TAT-D2R-KL (single intraperitoneal administration at 3 nmol/g in both cases). 1 hour after the treatment, the open field test (OFT), the forced swimming test (FST), the tail suspension test (TST) and the sucrose preference test (SPT) were performed.

The test methods were as follows.

OFT (for testing the total distance): The mice were acclimated to the experimental environment for 1 h and placed in a chamber of 45 cm $\times$ 45 cm $\times$ 30 cm. A video was recorded for 5 min to observe the motional activity of the mice. The total distance covered by the mice was measured, analyzed and expressed in millimeters.

FST: The mice were placed in a cylinder (having a height of 70 cm and a diameter of 30 cm) made of organic glass and filled with water (with water temperature at $23 \pm 1°C$), wherein the water level was more than 30 cm from the bottom. The mice were recorded in video for 5 min, then the video was analyzed, and the immobility time was recorded. Immobility was defined as no movement of a mouse floating in water or keeping its nose above the water surface. Swimming was defined as the horizontal movement over the whole cylinder. Climbing was defined as the vertical movement against the wall of the cylinder.

TST: The mice were suspended with a tape to a position 40 cm above the floor within a rectangular compartment (length 55 cm $\times$ width 20 cm $\times$ depth 11.5 cm). A video was recorded for a total of 5 minutes, and the immobility time was recorded. The EthoVisionXT software was used for recording and analysis.

SPT (Sucrose preference test): A two-bottle choice paradigm was adopted. The mice were habituated with 1% sucrose solution for 3 days and randomized into groups. To assess the sucrose intake of individual, the mice were deprived of water and food for 24 h over a 3-day period. The next day, each mouse was allowed to have free access to two bottles containing sucrose or water. After 2.5 hours, the positions of the bottles containing water or sucrose were changed. The test was carried out for a total of 5 hours. Finally, the volume of consumed water or sucrose solution was recorded and calculation was carried out with the following equation (I):

$$SPT=\frac{\text{consumption of sucrose}}{\text{consumption of water and sucrose}}\times100\% \quad (I)$$

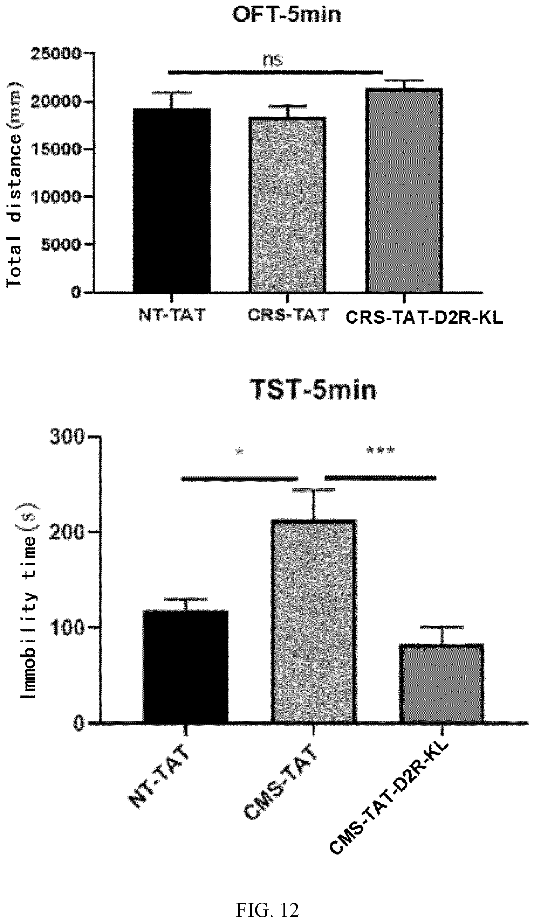

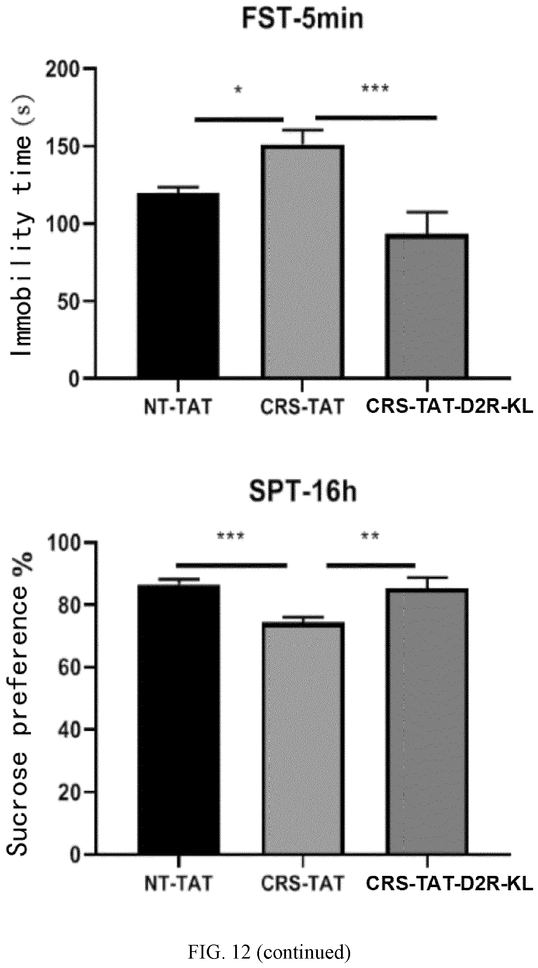

It could be seen from the analysis results in FIG. 12 that the TAT-D2R-KL treatment had no effect on the overall movement ability of the mice. Compared with the TAT-treated mice, the TAT-D2R-KL treated mice showed a significantly reduced immobility time, and significantly enhanced sucrose preference, demonstrating a significant anti-depression effect.

Example 7 Evaluation of the anti-anxiety ability of the polypeptide using a medicament evaluation model

In order to evaluate the anti-anxiety-like effect of the polypeptide of the present invention, a CRS mouse model was established using the method in Example 6. The mice were treated with TAT or TAT-D2R-KL (single intraperitoneal administration at 3 nmol/g in both cases), and 1 hour later, an open field test (OFT) for testing the time spent in the central area and an elevated plus maze (EPM) test were performed. The test methods were as follows.

OFT (for testing the time spent in the central area): The bottom of the open field was divided into 25 squares of equal area, with the 9 squares in the middle defined as the central area. One hour after the polypeptide treatment, the mice were placed in the central area to be recorded for 5 min, and the time spent in the central area by the mice was recorded.

EPM: The device consisted of two open arms (25 cm × 8 cm) and two closed arms (25 cm × 8 cm) with the intersection as the central area (8 cm × 8 cm), elevated 40 cm above the ground. One hour after the polypeptide treatment, the mice were placed in the central area facing an open arm, and their free movements were recorded for 5 min. After each animal was tested, the device was wiped with 70% alcohol. The time spent in an open arm, the time spent in a closed arm, and the total moving distance of the mice were recorded.

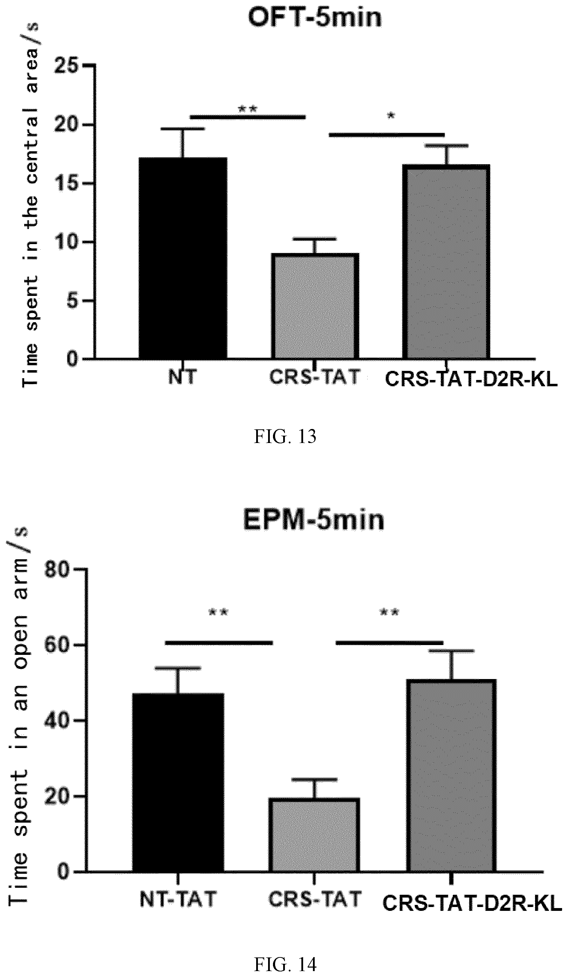

The OFT results were shown in FIG. 13, wherein the TAT-D2R-KL peptide significantly increased the time spent in the central area by the mice. The EPM results were shown in FIG. 14, wherein the TAT-D2R-KL peptide significantly increased the time spent in an open arm by the mice, demonstrating that the polypeptide of the present invention effectively reduced the anxious emotion.

**Claims**

1. Use of a polypeptide in preparation of a medicament for treating and/or preventing PTSD, depression, or anxiety disorder, wherein the polypeptide consists of up to 30 consecutive amino acid residues derived from human D2R and comprises the sequence shown in SEQ ID NO: 1.

2. The use according to claim 1, wherein the polypeptide consists of up to 25, preferably up to 20 consecutive amino acid residues derived from human D2R.

3. The use according to claim 1 or 2, wherein the amino acid sequence of the human D2R is as shown in SEQ ID NO: 2.

4. A polypeptide consisting of up to 30 consecutive amino acid residues derived from human D2R and comprising the sequence shown in SEQ ID NO: 1.

5. The polypeptide according to claim 4, wherein the polypeptide consists of up to 25, preferably up to 20 consecutive amino acid residues derived from human D2R.

6. The polypeptide according to claim 4 or 5, wherein the amino acid sequence of the human D2R is as shown in SEQ ID NO: 2.

7. A complex comprising the polypeptide according to any one of claims 4 to 6 and a carrier for permeating the blood-brain barrier linked to the polypeptide, preferably the carrier for permeating the blood-brain barrier is one or more selected from the group consisting of HIV-1 Tat protein, insulin, cationized albumin, mAb against rat transferrin receptor, human insulin receptor murine mAb, Penetratin, transduction domain of Tat protein, Pep-1 peptide, S4$_{13}$-PV, Magainin 2, and Buforin 2.

8. A nucleic acid molecule which:

    a) encodes the polypeptide according to any one of claims 4 to 6 or the complex according to claim 7;

b) is reverse-complement to the molecule according to a); or
c) is as shown in SEQ ID NO: 4.

**9.** An expression vector containing the nucleic acid molecule according to claim 8.

**10.** A host cell containing the nucleic acid molecule according to claim 8 or the expression vector according to claim 9.

FIG. 1

FIG. 2

FIG. 3

FIG.4

FIG.5

FIG. 6

FIG. 7

FIG. 8

FIG.9

FIG.10

FIG. 11

FIG. 12

## FST-5min

## SPT-16h

FIG. 12 (continued)

FIG. 13

FIG. 14

## INTERNATIONAL SEARCH REPORT

| International application No. |
| --- |
| **PCT/CN2023/089490** |

**A. CLASSIFICATION OF SUBJECT MATTER**

A61K 38/17(2006.01)i; A61P 25/00(2006.01)i; A61P 25/24(2006.01)i; A61P 25/22(2006.01)i; C07K 14/705(2006.01)i; C12N 15/12(2006.01)i; C12N 15/70(2006.01)i; C12N 15/85(2006.01)i; C12N 5/10(2006.01)i; C12N 1/21(2006.01)i

According to International Patent Classification (IPC) or to both national classification and IPC

**B. FIELDS SEARCHED**

Minimum documentation searched (classification system followed by classification symbols)

IPC:A61K,A61P,CO7K,C12N

Documentation searched other than minimum documentation to the extent that such documents are included in the fields searched

Electronic data base consulted during the international search (name of data base and, where practicable, search terms used)

CNTXT, WPABS, WPABSC, ENTXTC, CJFD, CNKI, 万方数据资源系统, Wanfang Data Resource System, PubMed, ScienceDirect, GenBank, EBI-EMBL, STN, 中国专利生物序列检索系统, China Patent Biological Sequence Search System: 申请人/发明人, 序列检索SEQ ID NOs: 1-2, 4, 多巴胺受体, D2受体, 胞内环3, 5-羟色胺受体, 5-羟色胺受体, 血清素受体, 异源受体聚体, 受体聚合, 复合物, 焦虑, 抑郁, 创伤后应激, DRD2, DR2, D2R, IC3, intracellular loop3, IL3, 5-ht, 5ht, Serotonin receptor, heteroreceptor complexes, receptor oligomerization, heterocomplex, PTSD, depression, anxiety.

**C. DOCUMENTS CONSIDERED TO BE RELEVANT**

| Category* | Citation of document, with indication, where appropriate, of the relevant passages | Relevant to claim No. |
| --- | --- | --- |
| PX | CN 114767832 A (SHENZHEN CHENYANG BIOTECHNOLOGY CO., LTD.) 22 July 2022 (2022-07-22)<br>   entire document | 1-10 |
| X | WO 02061087 A2 (LIFESPAN BIOSCIENCES, INC. et al.) 08 August 2002 (2002-08-08)<br>   claims 1-5 and 21-25 | 4-10 |
| A | US 2008213271 A1 (CENTER FOR ADDICTION AND MENTAL HEALTH) 04 September 2008 (2008-09-04)<br>   entire document | 1-10 |
| A | CN 113501881 A (PEKING UNIVERSITY) 15 October 2021 (2021-10-15)<br>   entire document | 1-10 |
| A | CN 113813364 A (SHENZHEN CHENYANG BIOTECHNOLOGY CO., LTD.) 21 December 2021 (2021-12-21)<br>   entire document | 1-10 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **21 July 2023** | **28 July 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/089490**

### C.    DOCUMENTS CONSIDERED TO BE RELEVANT

| Category* | Citation of document, with indication, where appropriate, of the relevant passages | Relevant to claim No. |
|---|---|---|
| A | CN 113855784 A (SHENZHEN CHENYANG BIOTECHNOLOGY CO., LTD.) 31 December 2021 (2021-12-31)<br>        entire document | 1-10 |
| A | BORROTO-ESCUELA,D.O. et al. "Multiple D2 heteroreceptor complexes: new targets for treatment of schizophrenia"<br>*THER.ADV.PSYCHOPHARMACOL.*, Vol. 6, No. (3), 10 March 2006 (2006-03-10),<br>        77-94 | 1-10 |

Form PCT/ISA/210 (second sheet) (July 2022)

**INTERNATIONAL SEARCH REPORT**

International application No.

**PCT/CN2023/089490**

| Box No. I | Nucleotide and/or amino acid sequence(s) (Continuation of item 1.c of the first sheet) |

1.   With regard to any nucleotide and/or amino acid sequence disclosed in the international application, the international search was carried out on the basis of a sequence listing:

    a.   ☑ forming part of the international application as filed.

    b.   ☐ furnished subsequent to the international filing date for the purposes of international search (Rule 13*ter*.1(a)),

        ☐ accompanied by a statement to the effect that the sequence listing does not go beyond the disclosure in the international application as filed.

2.   ☐ With regard to any nucleotide and/or amino acid sequence disclosed in the international application, this report has been established to the extent that a meaningful search could be carried out without a WIPO Standard ST.26 compliant sequence listing.

3.   Additional comments:

Form PCT/ISA/210 (continuation of first sheet) (July 2022)

EP 4 512 412 A1

## INTERNATIONAL SEARCH REPORT
### Information on patent family members

International application No.

**PCT/CN2023/089490**

| Patent document cited in search report | | | Publication date (day/month/year) | Patent family member(s) | | | Publication date (day/month/year) |
|---|---|---|---|---|---|---|---|
| CN | 114767832 | A | 22 July 2022 | None | | | |
| WO | 02061087 | A2 | 08 August 2002 | US | 2003113798 | A1 | 19 June 2003 |
| | | | | WO | 02061087 | A3 | 19 June 2003 |
| US | 2008213271 | A1 | 04 September 2008 | US | 2013345122 | A1 | 26 December 2013 |
| | | | | CA | 2607095 | A1 | 09 November 2006 |
| | | | | US | 2013035293 | A1 | 07 February 2013 |
| | | | | US | 8552152 | B2 | 08 October 2013 |
| | | | | US | 2011091467 | A1 | 21 April 2011 |
| | | | | US | 8304519 | B2 | 06 November 2012 |
| | | | | WO | 2006116874 | A1 | 09 November 2006 |
| CN | 113501881 | A | 15 October 2021 | CN | 109553687 | A | 02 April 2019 |
| | | | | WO | 2019062744 | A1 | 04 April 2019 |
| | | | | US | 2020400567 | A1 | 24 December 2020 |
| | | | | CN | 109553687 | B | 06 July 2021 |
| CN | 113813364 | A | 21 December 2021 | None | | | |
| CN | 113855784 | A | 31 December 2021 | WO | 2023045662 | A1 | 30 March 2023 |

Form PCT/ISA/210 (patent family annex) (July 2022)